# EUROPEAN PATENT SPECIFICATION

(11) **EP 3 606 487 B1**
(45) Date of publication and mention of the grant of the patent: **06.07.2022**
(21) Application number: 18719020.2
(22) Date of filing: 02.04.2018
(51) Int. Cl.: A61F 13/53, D21H 27/00, D21H 27/30, D21H 27/32, D21H 27/38, D21H 15/10

(54) **MULTI-LAYER UNITARY ABSORBENT STRUCTURES**
MEHRLAGIGE EINHEITLICHE SAUGFÄHIGE STRUKTUREN
STRUCTURES ABSORBANTES UNITAIRES MULTICOUCHES

(30) Priority: 03.04.2017 US 201762481000 P
(43) Date of publication of application: 12.02.2020
(73) Proprietor: Georgia-Pacific Mt. Holly LLC, Atlanta, GA 30303 (US)
(72) Inventor: DUTKIEWICZ, Jacek K., Cordova, Tennessee 38016 (US)
(74) Representative: Grünecker Patent- und Rechtsanwälte PartG mbB
(86) International application number: PCT/US2018/025627
(87) International publication number: WO 2018/187192

(56) References cited:
- WO-A1-03/039852
- WO-A1-2004/044328
- WO-A1-2015/176063
- US-A1- 2009 019 825

## Description

### 1. FIELD OF THE INVENTION

The presently disclosed subject matter relates to new absorbent structures and their use in articles including diapers and incontinence products, feminine hygiene products, and other consumer products such as cleaning products. More particularly, the presently disclosed subject matter relates to multi-layer unitary structures having improved fluid acquisition and retention characteristics.

### 2. BACKGROUND OF THE INVENTION

Nonwoven structures are important in a wide range of consumer products, such as absorbent articles including baby diapers, adult incontinence products, sanitary napkins, cleaning products, and the like. For example, many such consumer products have absorbent properties to collect and retain fluids. Such absorbent articles must simultaneously perform multiple functions, including liquid distribution from the point of liquid insult and storage of liquid within internal layers.

As such, absorbent materials often include an absorbent core sandwiched between a liquid pervious topsheet, whose function is to allow the passage of fluid to the core, and a liquid impervious backsheet, whose function is to contain the fluid and to prevent it from passing through the absorbent article to the garment of the wearer of the absorbent article. In recent years, market demand for an increasingly thinner and more comfortable absorbent article has increased. There has also been increased desire to reduce material costs and simplify manufacturing. However, to-date, most absorbent materials include the three distinct components described above, *i.e.,* the topsheet, absorbent core, and backsheet.

Thus, there remains a need for an absorbent material with a simpler construction that is able to provide the necessary thinness, comfort, and liquid acquisition and retention characteristics for use in absorbent articles, such as hygiene products. The disclosed subject matter addresses these needs.

US 2009/019825 A1 discloses a two-ply nonwoven structure with a first layer comprising a first type of bicomponent fiber and a second layer comprising a second type of bicomponent fiber.

### 3. SUMMARY

The presently disclosed subject matter provides for an absorbent structure formed from a unitary multi-layer nonwoven material containing specific layered constructions, which advantageously provides improved fluid acquisition and retention characteristics.

A first aspect of the present invention relates to a multi-layer unitary absorbent structure comprising a first layer comprising a first bicomponent fiber and having a basis weight from about 5 gsm to about 50 gsm and a second layer, adjacent to the first layer, comprising a second bicomponent fiber and having a basis weight from about 5 gsm to about 50 gsm, wherein the first bicomponent fiber is different than the second bicomponent fiber, as described in claim 1.

In certain embodiments, the multi-layer unitary absorbent structure can further include a third layer, adjacent to the second layer, comprising spunbond fibers. For example, the second bicomponent fiber can have a greater dtex than the first bicomponent fiber. Alternatively, the first bicomponent fiber can have a greater dtex than the second bicomponent fiber.

The first layer includes a blend of at least two different bicomponent fibers. For example, the at least two different bicomponent fibers can differ by at least one of composition, configuration, dtex, and length.

In certain embodiments, the multi-layer unitary absorbent structure can further include a cellulosic fiber layer adjacent to the second layer. Alternatively or additionally, the multi-layer unitary absorbent structure can further include an absorbent core. In certain embodiments, the absorbent core can include SAP. The multi-layer unitary absorbent structure can further include a binder on at least a portion of the external surface of at least one outer layer.

A second aspect of the present invention relates to an absorbent article comprising a multi-layer unitary absorbent structure as described above.

In certain embodiments, the absorbent article can be a hygiene product. For example, and not limitation, the hygiene product can be selected from a baby diaper, an adult incontinence product, and a sanitary napkin.

The foregoing has outlined broadly the features and technical advantages of the present application in order that the detailed description that follows may be better understood. Additional features and advantages of the application will be described hereinafter which form the subject of the claims of the application. It should be appreciated by those skilled in the art that the conception and specific embodiment disclosed may be readily utilized as a basis for modifying or designing other structures for carrying out the same purposes of the present application. It should also be realized by those skilled in the art that such equivalent constructions do not depart from the scope of the application as set forth in the appended claims. The novel features which are believed to be characteristic of the application, both as to its organization and method of operation, together with further objects and advantages will be better understood from the following description.

### 4. DETAILED DESCRIPTION

The presently disclosed subject matter provides multi-layer absorbent structures for use in absorbent articles. The presently disclosed subject matter also provides methods for making such materials. These and other aspects of the disclosed subject matter are discussed more in the detailed description and examples.

### Definitions

The terms used in this specification generally have their ordinary meanings in the art, within the context of this subject matter and in the specific context where each term is used. Certain terms are defined below to provide additional guidance in describing the compositions and methods of the disclosed subject matter and how to make and use them.

As used herein, a "nonwoven" refers to a class of material, including but not limited to textiles or plastics. Nonwovens are sheet or web structures made of fiber, filaments, molten plastic, or plastic films bonded together mechanically, thermally, or chemically. A nonwoven is a fabric made directly from a web of fiber, without the yarn preparation necessary for weaving or knitting. In a nonwoven, the assembly of fibers is held together by one or more of the following: (1) by mechanical interlocking in a random web or mat; (2) by fusing of the fibers, as in the case of thermoplastic fibers; or (3) by bonding with a cementing medium such as a natural or synthetic resin. As used herein, "nonwoven" refers to a variety of materials, including spunbond and melt blown materials.

As used herein, the term "liquid" refers to a substance having a fluid consistency. For example, and not limitation, liquids can include water, oils, solvents, bodily fluids such as urine or blood, wet foodstuff such as beverages and soups, disinfectants, lotions, and cleaning solutions.

As used herein, the term "weight percent" is meant to refer to either (i) the quantity by weight of a constituent/component in the material as a percentage of the weight of a layer of the material; or (ii) to the quantity by weight of a constituent/component in the material as a percentage of the weight of the final nonwoven material or product.

The term "basis weight" as used herein refers to the quantity by weight of a compound over a given area. Examples of the units of measure include grams per square meter as identified by the acronym "gsm".

As used in the specification and the appended claims, the singular forms "a," "an" and "the" include plural referents unless the context clearly dictates otherwise. Thus, for example, reference to "a compound" includes mixtures of compounds.

The term "about" or "approximately" means within an acceptable error range for the particular value as determined by one of ordinary skill in the art, which will depend in part on how the value is measured or determined, *i.e.*, the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to systems or processes, the term can mean within an order of magnitude, preferably within 5-fold, and more preferably within 2-fold, of a value.

### Fibers

The absorbent structures of the presently disclosed subject matter can be nonwoven materials comprising fibers. The fibers can be natural, synthetic, or a mixture thereof. In one embodiment, the fibers can include one or more synthetic fibers, or a mixture thereof.

### Synthetic Fibers

In addition to the use of cellulose fibers, the presently disclosed subject matter also contemplates the use of synthetic fibers. In one embodiment, the synthetic fibers comprise bicomponent and/or mono-component fibers. Bicomponent fibers having a core and sheath are known in the art. Many varieties are used in the manufacture of nonwoven materials, particularly those produced for use in airlaid techniques. Various bicomponent fibers suitable for use in the presently disclosed subject matter are disclosed in U.S. Patent Nos. 5,372,885 and 5,456,982 Examples of bicomponent fiber manufacturers include, but are not limited to, Trevira (Bobingen, Germany), Fiber Innovation Technologies (Johnson City, TN) and ES Fiber Visions (Athens, GA).

Bicomponent fibers can incorporate a variety of polymers as their core and sheath components. Bicomponent fibers that have a PE (polyethylene) or modified PE sheath typically have a PET (polyethylene terephthalate) or PP (polypropylene) core. In one embodiment, the bicomponent fiber has a core made of polypropylene and sheath made of polyethylene.

The denier of the bicomponent fiber preferably ranges from about 1.0 dpf to about 4.0 dpf, and more preferably from about 1.5 dpf to about 2.5 dpf The length of the bicomponent fiber can be from about 3 mm to about 36 mm, preferably from about 3 mm to about 12 mm, more preferably from about 3 mm to about 10. In particular embodiments, the length of the bicomponent fiber is from about 4 mm to about 8 mm, or about 4 mm to about 6 mm. In a particular embodiment, the bicomponent fiber is Trevira T255 which contains a polyester core and a polyethylene sheath modified with maleic anhydride. T255 has been produced in a variety of deniers, cut lengths and core sheath configurations with preferred configurations having a denier from about 1.7 dpf to 2.0 dpf and a cut length of about 4 mm to 12 mm and a concentric core sheath configuration. In a specific embodiment, the bicomponent fiber is Trevira T255, 1.7 dtex and 6 mm in length. In an alternate embodiment, the bicomponent fiber is an eccentric bicomponent fiber with a polypropylene core and a polyethylene sheath, such as Fibervisions ESE430ALV1, 3.3 dtex and 4 mm in length or Fibervisions ESE452ALV1, 5.7 dtex and 4 mm in length.

Bicomponent fibers are typically fabricated commercially by melt spinning. In this procedure, each molten polymer is extruded through a die, for example, a spinneret, with subsequent pulling of the molten polymer to move it away from the face of the spinneret. This is followed by solidification of the polymer by heat transfer to a surrounding fluid medium, for example chilled air, and taking up of the now solid filament. Non-limiting examples of additional steps after melt spinning can also include hot or cold drawing, heat treating, crimping and cutting. This overall manufacturing process is generally carried out as a discontinuous two-step process that first involves spinning of the filaments and their collection into a tow that comprises numerous filaments. During the spinning step, when molten polymer is pulled away from the face of the spinneret, some drawing of the filament does occur which can also be called the draw-down. This is followed by a second step where the spun fibers are drawn or stretched to increase molecular alignment and crystallinity and to give enhanced strength and other physical properties to the individual filaments. Subsequent steps can include, but are not limited to, heat setting, crimping and cutting of the filament into fibers. The drawing or stretching step can involve drawing the core of the bicomponent fiber, the sheath of the bicomponent fiber or both the core and the sheath of the bicomponent fiber depending on the materials from which the core and sheath are comprised as well as the conditions employed during the drawing or stretching process.

Bicomponent fibers can also be formed in a continuous process where the spinning and drawing are done in a continuous process. During the fiber manufacturing process it is desirable to add various materials to the fiber after the melt spinning step at various subsequent steps in the process. These materials can be referred to as "finish" and be comprised of active agents such as, but not limited to, lubricants and anti-static agents. The finish is typically delivered via an aqueous based solution or emulsion. Finishes can provide desirable properties for both the manufacturing of the bicomponent fiber and for the user of the fiber, for example in an airlaid or wetlaid process.

Numerous other processes are involved before, during and after the spinning and drawing steps and are disclosed in U.S. Patent Nos. 4,950,541, 5,082,899, 5,126,199, 5,372,885, 5,456,982, 5,705,565, 2,861,319, 2,931,091, 2,989,798, 3,038,235, 3,081,490, 3,117,362, 3,121,254, 3,188,689, 3,237,245, 3,249,669, 3,457,342, 3,466,703, 3,469,279, 3,500,498, 3,585,685, 3,163,170, 3,692,423, 3,716,317, 3,778,208, 3,787,162, 3,814,561, 3,963,406, 3,992,499, 4,052,146, 4,251,200, 4,350,006, 4,370,114, 4,406,850, 4,445,833, 4,717,325, 4,743,189, 5,162,074, 5,256,050, 5,505,889, 5,582,913, and 6,670,035.

The presently disclosed subject matter can also include, but are not limited to, articles that contain bicomponent fibers that are partially drawn with varying degrees of draw or stretch, highly drawn bicomponent fibers and mixtures thereof. These can include, but are not limited to, a highly drawn polyester core bicomponent fiber with a variety of sheath materials, specifically including a polyethylene sheath such as Trevira T255 (Bobingen, Germany) or a highly drawn polypropylene core bicomponent fiber with a variety of sheath materials, specifically including a polyethylene sheath such as ES FiberVisions AL-Adhesion-C (Varde, Denmark). Additionally, Trevira T265 bicomponent fiber (Bobingen, Germany), having a partially drawn core with a core made of polybutylene terephthalate (PBT) and a sheath made of polyethylene can be used. The use of both partially drawn and highly drawn bicomponent fibers in the same structure can be leveraged to meet specific physical and performance properties based on how they are incorporated into the structure.

The bicomponent fibers of the presently disclosed subject matter are not limited in scope to any specific polymers for either the core or the sheath as any partially drawn core bicomponent fiber can provide enhanced performance regarding elongation and strength. The degree to which the partially drawn bicomponent fibers are drawn is not limited in scope as different degrees of drawing will yield different enhancements in performance. The scope of the partially drawn bicomponent fibers encompasses fibers with various core sheath configurations including, but not limited to concentric, eccentric, side by side, islands in a sea, pie segments and other variations. The relative weight percentages of the core and sheath components of the total fiber can be varied. In addition, the scope of this subject matter covers the use of partially drawn homopolymers such as polyester, polypropylene, nylon, and other melt spinnable polymers. The scope of this subject matter also covers multicomponent fibers that can have more than two polymers as part of the fiber structure.

In particular embodiments, the bicomponent fibers in a particular layer comprise from about 50 to about 100 percent by weight of the layer. The bicomponent layer can contain from about 1 gsm to about 50 gsm bicomponent fibers, or about 1 gsm to about 40 gsm bicomponent fibers, or about 1 gsm to about 30 gsm bicomponent fibers, or from about 2 gsm to about 20 gsm bicomponent fibers, or about 10 gsm to about 20 gsm bicomponent fibers. In certain embodiments, the bicomponent layer contains from about 4 gsm to about 20 gsm bicomponent fibers. In alternative embodiments, the bicomponent layer contains from about 10 gsm to about 50 gsm bicomponent fibers, or from about 12 gsm to about 40 gsm bicomponent fibers, or from about 20 gsm to about 30 gsm bicomponent fibers.

In particular embodiments, the bicomponent fibers are low dtex staple bicomponent fibers in the range of about 0.5 dtex to about 20 dtex. In certain embodiments, the dtex value can range from about 1 dtex to about 15 dtex, or from about 1.5 dtex to about 10 dtex, or from about 1.7 dtex to about 5.7 dtex. In certain embodiments, the dtex value is 1.7 dtex, 2.2 dtex, 3.3 dtex, 5.7 dtex, 6.7 dtex, or 10 dtex.

Other synthetic fibers suitable for use in various embodiments as fibers or as bicomponent binder fibers include, but are not limited to, fibers made from various polymers including, by way of example and not by limitation, acrylic, polyamides (including, but not limited to, Nylon 6, Nylon 6/6, Nylon 12, polyaspartic acid, polyglutamic acid), polyamines, polyimides, polyacrylics (including, but not limited to, polyacrylamide, polyacrylonitrile, esters of methacrylic acid and acrylic acid), polycarbonates (including, but not limited to, polybisphenol A carbonate, polypropylene carbonate), polydienes (including, but not limited to, polybutadiene, polyisoprene, polynorbomene), polyepoxides, polyesters (including, but not limited to, polyethylene terephthalate, polybutylene terephthalate, polytrimethylene terephthalate, polycaprolactone, polyglycolide, polylactide, polyhydroxybutyrate, polyhydroxyvalerate, polyethylene adipate, polybutylene adipate, polypropylene succinate), polyethers (including, but not limited to, polyethylene glycol (polyethylene oxide), polybutylene glycol, polypropylene oxide, polyoxymethylene (paraformaldehyde), polytetramethylene ether (polytetrahydrofuran), polyepichlorohydrin), polyfluorocarbons, formaldehyde polymers (including, but not limited to, urea-formaldehyde, melamine-formaldehyde, phenol formaldehyde), natural polymers (including, but not limited to, cellulosics, chitosans, lignins, waxes), polyolefins (including, but not limited to, polyethylene, polypropylene, polybutylene, polybutene, polyoctene), polyphenylenes (including, but not limited to, polyphenylene oxide, polyphenylene sulfide, polyphenylene ether sulfone), silicon containing polymers (including, but not limited to, polydimethyl siloxane, polycarbomethyl silane), polyurethanes, polyvinyls (including, but not limited to, polyvinyl butyral, polyvinyl alcohol, esters and ethers of polyvinyl alcohol, polyvinyl acetate, polystyrene, polymethylstyrene, polyvinyl chloride, polyvinyl pryrrolidone, polymethyl vinyl ether, polyethyl vinyl ether, polyvinyl methyl ketone), polyacetals, polyarylates, and copolymers (including, but not limited to, polyethylene-co-vinyl acetate, polyethylene-co-acrylic acid, polybutylene terephthalate-co-polyethylene terephthalate, polylauryllactam-block-polytetrahydrofuran), polybutylene succinate and polylactic acid based polymers.

In specific embodiments, the synthetic fiber layer contains a high dtex staple fibers in the range of about 2 to about 20 dtex. In certain embodiments, the dtex value can range from about 2 dtex to about 15 dtex, or from about 2 dtex to about 10 dtex. In particular embodiments, the fiber can have a dtex value of about 6.7 dtex.

In other specific embodiments, the synthetic layer contains synthetic filaments. The synthetic filaments can be formed by spinning and/or extrusion processes. For example, such processes can be similar to the methods described above with reference to melt spinning processes. The synthetic filaments can include one or more continuous strands. In certain embodiments, the synthetic filaments can include polypropylene.

In particular embodiments, polyester (PET) fibers such as Trevira Type 245, are used in a synthetic fiber layer comprising from about 50 to about 100 percent by weight of the layer. The synthetic fiber layer contains from about 5 gsm to about 50 gsm synthetic fibers, or from about 10 gsm to about 40 gsm synthetic fibers, or from about 20 to about 30 synthetic fibers, or about 30 gsm synthetic fibers.

### Cellulose Fibers

Any cellulose fibers known in the art, including cellulose fibers of any natural origin, such as those derived from wood pulp or regenerated cellulose, can be used in a cellulosic layer. In certain embodiment, cellulose fibers include, but are not limited to, digested fibers, such as kraft, prehydrolyzed kraft, soda, sulfite, chemi-thermal mechanical, and thermo-mechanical treated fibers, derived from softwood, hardwood or cotton linters. In other embodiments, cellulose fibers include, but are not limited to, kraft digested fibers, including prehydrolyzed kraft digested fibers. Non-limiting examples of cellulose fibers suitable for use in this subject matter are the cellulose fibers derived from softwoods, such as pines, firs, and spruces. Other suitable cellulose fibers include, but are not limited to, those derived from Esparto grass, bagasse, kemp, flax, hemp, kenaf, and other lignaceous and cellulosic fiber sources. Suitable cellulose fibers include, but are not limited to, bleached Kraft southern pine fibers sold under the trademark FOLEY FLUFFS^{®} (Buckeye Technologies Inc., Memphis, Tenn.). Additionally, fibers sold under the trademark CELLU TISSUE^{®} (*e*.*g*., Grade 3024) (Clearwater Paper Corporation, Spokane, Wash.) are utilized in certain aspects of the disclosed subject matter.

The absorbent structures of the disclosed subject matter can also include, but are not limited to, a commercially available bright fluff pulp including, but not limited to, southern softwood fluff pulp (such as Treated FOLEY FLUFFS^{®}) northern softwood sulfite pulp (such as T 730 from Weyerhaeuser), or hardwood pulp (such as eucalyptus). While certain pulps may be preferred based on a variety of factors, any absorbent fluff pulp or mixtures thereof can be used. In certain embodiments, wood cellulose, cotton linter pulp, chemically modified cellulose such as crosslinked cellulose fibers and highly purified cellulose fibers can be used. Non-limiting examples of additional pulps are FOLEY FLUFFS^{®} FFTAS (also known as FFTAS or Buckeye Technologies FFT-AS pulp), and Weyco CF401.

Other suitable types of cellulose fiber include, but are not limited to, chemically modified cellulose fibers. In particular embodiments, the modified cellulose fibers are crosslinked cellulose fibers. U.S. Patent Nos. 5,492,759; 5,601,921; 6,159,335, relate to chemically treated cellulose fibers useful in the practice of this disclosed subject matter. In certain embodiments, the modified cellulose fibers comprise a polyhydroxy compound. Non-limiting examples of polyhydroxy compounds include glycerol, trimethylolpropane, pentaerythritol, polyvinyl alcohol, partially hydrolyzed polyvinyl acetate, and fully hydrolyzed polyvinyl acetate. In certain embodiments, the fiber is treated with a polyvalent cation-containing compound. In one embodiment, the polyvalent cation-containing compound is present in an amount from about 0.1 weight percent to about 20 weight percent based on the dry weight of the untreated fiber. In particular embodiments, the polyvalent cation containing compound is a polyvalent metal ion salt. In certain embodiments, the polyvalent cation containing compound is selected from the group consisting of aluminum, iron, tin, salts thereof, and mixtures thereof. Any polyvalent metal salt including transition metal salts may be used. Non-limiting examples of suitable polyvalent metals include beryllium, magnesium, calcium, strontium, barium, titanium, zirconium, vanadium, chromium, molybdenum, tungsten, manganese, iron, cobalt, nickel, copper, zinc, aluminum and tin. Preferred ions include aluminum, iron and tin. The preferred metal ions have oxidation states of +3 or +4. Any salt containing the polyvalent metal ion may be employed. Non-limiting examples of suitable inorganic salts of the above metals include chlorides, nitrates, sulfates, borates, bromides, iodides, fluorides, nitrides, perchlorates, phosphates, hydroxides, sulfides, carbonates, bicarbonates, oxides, alkoxides phenoxides, phosphites, and hypophosphites. Non-limiting examples of suitable organic salts of the above metals include formates, acetates, butyrates, hexanoates, adipates, citrates, lactates, oxalates, propionates, salicylates, glycinates, tartrates, glycolates, sulfonates, phosphonates, glutamates, octanoates, benzoates, gluconates, maleates, succinates, and 4,5-dihydroxy-benzene-1,3-disulfonates. In addition to the polyvalent metal salts, other compounds such as complexes of the above salts include, but are not limited to, amines, ethylenediaminetetra-acetic acid (EDTA), diethylenetriaminepenta-acetic acid (DIPA), nitrilotri-acetic acid (NTA), 2,4-pentanedione, and ammonia may be used.

In one embodiment, the cellulose pulp fibers are chemically modified cellulose pulp fibers that have been softened or plasticized to be inherently more compressible than unmodified pulp fibers. The same pressure applied to a plasticized pulp web will result in higher density than when applied to an unmodified pulp web. Additionally, the densified web of plasticized cellulose fibers is inherently softer than a similar density web of unmodified fiber of the same wood type. Softwood pulps may be made more compressible using cationic surfactants as debonders to disrupt interfiber associations. Use of one or more debonders facilitates the disintegration of the pulp sheet into fluff in the airlaid process. Examples of debonders include, but are not limited to, those disclosed in U.S. Patent Nos. 4,432,833, 4,425,186 and 5,776,308.

One example of a debonder-treated cellulose pulp is FFLE+. Plasticizers for cellulose, which can be added to a pulp slurry prior to forming wetlaid sheets, can also be used to soften pulp, although they act by a different mechanism than debonding agents. Plasticizing agents act within the fiber, at the cellulose molecule, to make flexible or soften amorphous regions. The resulting fibers are characterized as limp. Since the plasticized fibers lack stiffness, the comminuted pulp is easier to densify compared to fibers not treated with plasticizers. Plasticizers include, but are not limited to, polyhydric alcohols such as glycerol, low molecular weight polyglycol such as polyethylene glycols, and polyhydroxy compounds. These and other plasticizers are described and exemplified in U.S. Pat. Nos. 4,098,996, 5,547,541 and 4,731,269.

Ammonia, urea, and alkylamines are also known to plasticize wood products, which mainly contain cellulose (A. J. Stamm, Forest Products Journal 5(6):413, 1955)

In particular embodiments of the disclosed subject matter, the following cellulose is used: GP4723, a fully treated pulp (available from Georgia-Pacific); GP4725, a semi-treated pulp (available from Georgia-Pacific); Tencel (available from Lenzing); cellulose flax fibers; Danufil (available from Kelheim); Viloft (available from Kelheim); GP4865, an odor control semi-treated pulp (available from Georgia-Pacific); Grade 3024 Cellu Tissue (available from Clearwater); Brawny Industrial Flax 500 (available from Georgia-Pacific).

In certain embodiments, a particular layer can contain from about 5 gsm to about 150 gsm cellulose fibers, or from about 5 gsm to about 100 gsm cellulose fibers, or from about 10 gsm to about 50 gsm cellulose fibers. In particular embodiments, a layer can contain from about 7 gsm to about 40 gsm cellulose fibers, or from about 10 gsm to about 30 gsm cellulose fibers, or from about 15 gsm to about 25 gsm cellulose fibers, or about 20 gsm cellulose fibers.

### Binders

Suitable binders include, but are not limited to, liquid binders and powder binders. Non-limiting examples of liquid binders include emulsions, solutions, or suspensions of binders. Non-limiting examples of binders include polyethylene powders, copolymer binders, vinylacetate ethylene binders, styrene-butadiene binders, urethanes, urethane-based binders, acrylic binders, thermoplastic binders, natural polymer based binders, and mixtures thereof.

Suitable binders include, but are not limited to, copolymers, vinylacetate ethylene ("VAE") copolymers, which can have a stabilizer such as Wacker Vinnapas 192, Wacker Vinnapas EF 539, Wacker Vinnapas EP907, Wacker Vinnapas EP129, Celanese Duroset E130, Celanese Dur-O-Set Elite 130 25-1813 and Celanese Dur-O-Set TX-849, Celanese 75-524A, polyvinyl alcohol-polyvinyl acetate blends such as Wacker Vinac 911, vinyl acetate homopolyers, polyvinyl amines such as BASF Luredur, acrylics, cationic acrylamides, polyacryliamides such as Bercon Berstrength 5040 and Bercon Berstrength 5150, hydroxyethyl cellulose, starch such as National Starch CATO RTM 232, National Starch CATO RTM 255, National Starch Optibond, National Starch Optipro, or National Starch OptiPLUS, guar gum, styrene-butadienes, urethanes, urethane-based binders, thermoplastic binders, acrylic binders, and carboxymethyl cellulose such as Hercules Aqualon CMC. In certain embodiments, the binder is a natural polymer based binder. Non-limiting examples of natural polymer based binders include polymers derived from starch, cellulose, chitin, and other polysaccharides.

In certain embodiments, the binder is water-soluble. In one embodiment, the binder is a vinylacetate ethylene copolymer. One non-limiting example of such copolymers is EP907 (Wacker Chemicals, Munich, Germany). Vinnapas EP907 can be applied at a level of about 10% solids incorporating about 0.75% by weight Aerosol OT (Cytec Industries, West Paterson, N.J.), which is an anionic surfactant. Other classes of liquid binders such as styrene-butadiene and acrylic binders can also be used.

In certain embodiments, the binder is not water-soluble. Examples of these binders include, but are not limited to, Vinnapas 124 and 192 (Wacker), which can have an opacifier and whitener, including, but not limited to, titanium dioxide, dispersed in the emulsion. Other binders include, but are not limited to, Celanese Emulsions (Bridgewater, N.J.) Elite 22 and Elite 33.

In certain embodiments, the binder is a thermoplastic binder. Such thermoplastic binders include, but are not limited to, any thermoplastic polymer which can be melted at temperatures which will not extensively damage the cellulose fibers. Preferably, the melting point of the thermoplastic binding material will be less than about 175°C. Examples of suitable thermoplastic materials include, but are not limited to, suspensions of thermoplastic binders and thermoplastic powders. In particular embodiments, the thermoplastic binding material can be, for example, polyethylene, polypropylene, polyvinylchloride, and/or polyvinylidene chloride.

In particular embodiments, the vinylacetate ethylene binder is non-crosslinkable. In one embodiment, the vinylacetate ethylene binder is crosslinkable. In certain embodiments, the binder is WD4047 urethane-based binder solution supplied by HB Fuller. In one embodiment, the binder is Michem Prime 4983-45N dispersion of ethylene acrylic acid ("EAA") copolymer supplied by Michelman. In certain embodiments, the binder is Dur-O-Set Elite 22LV emulsion of VAE binder supplied by Celanese Emulsions (Bridgewater, N.J.). As noted above, in particular embodiments, the binder is crosslinkable. It is also understood that crosslinkable binders are also known as permanent wet strength binders. A permanent wet-strength binder includes, but is not limited to, Kymene^{®} (Hercules Inc., Wilmington, Del.), Parez^{®} (American Cyanamid Company, Wayne, N.J.), Wacker Vinnapas or AF192 (Wacker Chemie AG, Munich, Germany), or the like. Various permanent wet-strength agents are described in U.S. Patent No. 2,345,543, U.S. Patent No. 2,926,116, and U.S. Patent No. 2,926,154.

Other permanent wet-strength binders include, but are not limited to, polyamine-epichlorohydrin, polyamide epichlorohydrin or polyamide-amine epichlorohydrin resins, which are collectively termed "PAE resins". Non-limiting exemplary permanent wet-strength binders include Kymene 557H or Kymene 557LX (Hercules Inc., Wilmington, Del.) and have been described in U.S. Patent No. 3,700,623 and U.S. Patent No. 3,772,076.

Alternatively, in certain embodiments, the binder is a temporary wet-strength binder. The temporary wet-strength binders include, but are not limited to, Hercobond^{®} (Hercules Inc., Wilmington, Del.), Parez^{®} 750 (American Cyanamid Company, Wayne, N.J.), Parez^{®} 745 (American Cyanamid Company, Wayne, N.J.), or the like. Other suitable temporary wet-strength binders include, but are not limited to, dialdehyde starch, polyethylene imine, mannogalactan gum, glyoxal, and dialdehyde mannogalactan. Other suitable temporary wet-strength agents are described in U.S. Patent No. 3,556,932, U.S. Patent No. 5,466,337, U.S. Patent No. 3,556,933, U.S. Patent No. 4,605,702, U.S. Patent No. 4,603,176, U.S. Patent No. 5,935,383, and U.S. Patent No. 6,017,417,

In certain embodiments, binders are applied as emulsions in amounts ranging from about 1 gsm to about 4 gsm, or from about 1.3 gsm to about 2.8 gsm, or from about 2 gsm to about 3 gsm. Binder can be applied to one side of a fibrous layer, preferably an externally facing layer. Alternatively, binder can be applied to both sides of a layer, in equal or disproportionate amounts.

### Other Additives

The materials of the presently disclosed subject matter can also contain other additives. For example, the materials can contain superabsorbent polymer (SAP). The types of superabsorbent polymers which may be used in the disclosed subject matter include, but are not limited to, SAPs in their particulate form or fibrous form, such as powder, irregular granules, spherical particles, staple fibers, and other elongated particles. U.S. Patent Nos. 5,147,343; 5,378,528; 5,795,439; 5,807,916; 5,849,211, and 6,403,857, describe various superabsorbent polymers and methods of making superabsorbent polymers. One example of a superabsorbent polymer forming system is crosslinked acrylic copolymers of metal salts of acrylic acid and acrylamide or other monomers such as 2-acrylamido-2-methylpropanesulfonic acid. Many conventional granular superabsorbent polymers are based on poly(acrylic acid) which has been crosslinked during polymerization with any of a number of multi-functional co-monomer crosslinking agents well-known in the art. Examples of multi-functional crosslinking agents are set forth in U.S. Patent Nos. 2,929,154; 3,224,986; 3,332,909; 4,076,673

For instance, crosslinked carboxylated polyelectrolytes can be used to form superabsorbent polymers. Other water-soluble polyelectrolyte polymers are known to be useful for the preparation of superabsorbents by crosslinking, these polymers include: carboxymethyl starch, carboxymethyl cellulose, chitosan salts, gelatine salts, etc. They are not, however, commonly used on a commercial scale to enhance absorbency of dispensable absorbent articles mainly due to their higher cost. Superabsorbent polymer granules useful in the practice of this subject matter are commercially available from a number of manufacturers, such as BASF, Dow Chemical (Midland, Mich.), Stockhausen (Greensboro, N.C.), Chemdal (Arlington Heights, Ill.), and Evonik (Essen, Germany). Non-limiting examples of SAP include a surface crosslinked acrylic acid based powder such as Stockhausen 9350 or SX70, BASF HySorb FEM 33N, or Evonik Favor SXM 7900.

In certain embodiments, SAP can be used in a layer in amounts ranging from about 5% to about 50% based on the total weight of the structure. In certain embodiments, the amount of SAP in a layer can range from about 10 gsm to about 50 gsm, or from about 12 gsm to about 40 gsm, or from about 15 gsm to about 25 gsm.

### Absorbent Structures

The presently disclosed subject matter provides for improved absorbent structures with many advantages over various commercially available materials. The presently disclosed materials have a unitary structure, with an ability to achieve comparable or improved overall absorbency performance to composite materials. The absorbency performance is measured by better fluid acquisition or improved rewet characteristics, while providing a slim profile and comfort for hygiene products.

The presently disclosed subject matter provides for a unitary absorbent structure. In certain embodiments, the absorbent structure includes at least two layers, at least three layers, at least four layers, at least five layers, or at least six layers, wherein each layer comprises a specific fibrous content.

In certain embodiments, the absorbent structure can include at least two synthetic fiber layers. For example the first synthetic fiber layer can contain a first type of bicomponent fibers and the second synthetic fiber layer can contain a second type of bicomponent fibers. In certain embodiments, the bicomponent fibers of the first synthetic fiber layer can be different than those of the second synthetic fiber layer. In certain embodiments, the bicomponent fibers of one or more layers can comprise a blend of different bicomponent fibers. Alternatively, the first synthetic fiber layer can contain polyester fibers and the second synthetic fiber layer can contain bicomponent fibers. In certain embodiments, one or more synthetic fiber layers can be a spunbond layer, *e.g.,* containing bicomponent fibers. In particular embodiments, the first synthetic fiber layer can contain polyester fibers (*e.g.,* PET) and the second synthetic fiber layer can contain spunbond bicomponent fibers. As such, the first synthetic fiber layer can provide a liquid barrier as an alternative to a conventional topsheet.

Alternatively, in particular embodiments, the absorbent structure can include at least three synthetic fiber layers. For example, a three-layer material can include a first synthetic fiber layer containing a first type of bicomponent fibers, a second synthetic fiber layer containing a second type of bicomponent fibers, and a third synthetic fiber layer containing spunbond fibers. Alternatively, a three-layer material can include three synthetic fiber layers, each containing one or more different bicomponent fibers.

One or more synthetic fiber layers containing bicomponent fibers include a blend of different bicomponent fibers. For example, and not limitation, the bicomponent fibers forming a blend in a particular synthetic layer can differ by composition, configuration, dtex, and/or fiber length. For instance, and without limitation, if two different bicomponent fibers are present in a blend, they can be present in a ratio of about 5:1 to about 1:5, or from about 4:1 to about 1:4.

In specific embodiments, the absorbent structure can be a two-layer material with two synthetic fiber layers. The first synthetic fiber layer includes a blend of at least two different bicomponent fibers. For example, the blend can include bicomponent fibers having two different compositions, *e*.*g*., a polyester core with a polyethylene sheath and a polypropylene core with a polyethylene sheath. One or both of the bicomponent fibers can have an eccentric core-sheath configuration. The second synthetic fiber layer can include a third type of bicomponent fibers. According to the present invention, the bicomponent fibers of the second synthetic fiber layer have a greater dtex than the bicomponent fibers of the blend forming the first synthetic fiber layer.

In particular embodiments, the first synthetic fiber layer of the two-layer material can include bicomponent fibers having a polyester core with a polyethylene sheath with a dtex of about 1.7 and bicomponent fibers having a polypropylene core with a polyethylene sheath with a dtex of about 2.2. The second synthetic fiber layer can include bicomponent fibers having a polypropylene core with a polyethylene sheath with a dtex of about 5.7. The length of the bicomponent fibers can range from about 3 mm to about 36 mm, preferably from about 3 mm to about 12 mm, or about 4 mm to about 6 mm.

In certain embodiments, the absorbent structure can be a three-layer material with three synthetic fiber layers, where the first synthetic fiber layer includes a blend of at least two different bicomponent fibers. The second synthetic fiber layer can include a third type of bicomponent fibers and the third synthetic fiber layer can include a fourth type of bicomponent fibers. In certain embodiments, the bicomponent fibers of the second and third synthetic fiber layer can have a greater dtex than the bicomponent fibers of the blend forming the first synthetic fiber layer.

As embodied herein, the specific arrangement of different bicomponent fibers in the various synthetic fiber layers can form a large-pore fibrous network. In certain embodiments, the different bicomponent fibers can establish multiple layers of bonded fibers forming a pore-size gradient with larger pores disposed closer to the point of insult by a fluid.

As embodied herein, any of the multi-layer absorbent structures described herein can further include an additional cellulosic fiber layer. The cellulosic fiber layer can be position at various locations within the multi-layer structures. For example, in certain embodiments, the cellulosic fiber layer can be positioned below two or more synthetic fiber layers. In certain embodiments, a cellulosic fiber layer can comprises bonded cellulose fluff or cellulosic tissue.

Further, and as embodied herein, one or more layers can be bonded on at least a portion of their outer surface with a binder, *e.g.,* a polymeric binder. It is not necessary that the binder chemically bond with a portion of the layer, although it is preferred that the binder remain associated in close proximity with the layer, by coating, adhering, precipitation, or any other mechanism such that it is not dislodged from the layer during normal handling of the layer. For convenience, the association between the layer and the binder discussed above can be referred to as the bond, and the compound can be said to be bonded to the layer.

In certain embodiments, the first synthetic fiber layer can contain from about 5 gsm to about 50 gsm, or from about 10 gsm to about 30 gsm, or from about 20 gsm to about 25 gsm of synthetic fibers. The second synthetic fiber layer can contain from about 5 gsm to about 50 gsm, or from about 10 gsm to about 20 gsm of synthetic fibers. The third synthetic fiber layer, if present, can contain from about 5 gsm to about 50 gsm, or from about 5 gsm to about 30 gsm, or from about 8 gsm to about 20 gsm of synthetic fibers. The cellulosic fiber layer, if present, can contain from about 5 gsm to about 50 gsm, or from about 10 gsm to about 30 gsm, or from about 20 gsm to about 25 gsm of cellulosic fibers. The binder can be applied to one or more outer layers in amounts ranging from about 1 gsm to about 10 gsm, or from about 2 gsm to about 8 gsm, or from about 3 gsm to about 6 gsm, or about 5 gsm.

### Absorbent Cores

In another aspect, the presently disclosed subject matter provides for a multi-layer absorbent structure that is adjacent to an absorbent core. The absorbent core can include at least one layer comprising a superabsorbent material, such as SAP. For example, and not limitation, the SAP can be present in particulate and/or fibrous form. In particular embodiments, the absorbent core can be a multi-bonded airlaid (MBAL) material containing SAP.

In other particular embodiments, the absorbent core has at least five layers, wherein each layer has a specific fibrous content. In certain embodiments, the first layer contains cellulose fibers, the second layer contains SAP, the third layer contains cellulose fibers, the fourth layer contains SAP, and the fifth layer contains cellulose fibers. In certain embodiments, one or more of the first layer, third layer, and/or fifth layer can further include bicomponent fibers. In certain embodiments, the nonwoven material can further include at least one additional layer adjacent to the absorbent core. In particular embodiments, the additional layer contains synthetic fibers.

In a specific embodiment, the first layer of the absorbent core contains from about 5 gsm to about 100 gsm, or from about 10 gsm to about 50 gsm of cellulose fibers. In certain embodiments, the second layer contains from about 10 gsm to about 50 gsm, or from about 12 gsm to about 40 gsm, or from about 15 gsm to about 25 gsm of SAP particles. In certain embodiments, the third layer contains from about 5 gsm to about 100 gsm, or from about 10 gsm to about 50 gsm cellulose fibers. In certain embodiments, the fourth layer contains from about 10 gsm to about 50 gsm, or from about 12 gsm to about 40 gsm, or from about 15 gsm to about 25 gsm of SAP particles. In certain embodiments, the fifth layer contains from about 5 gsm to about 100 gsm, or from about 10 gsm to about 50 gsm cellulose fibers. In certain embodiments, the cellulose fibers can be cellulose pulp. For example and not limitation, the cellulose fibers can be a hardwood pulp, such as eucalyptus pulp.

### Features of the Absorbent Structures

The presently disclosed unitary absorbent structures are able to provide a variety of different functions. For example, the absorbent structures provide a liquid barrier to retain liquids within the absorbent structure and can quickly absorb fluid insults. Moreover, the absorbent structures can provide both temporary and long-term fluid storage, particularly when used with an absorbent core. The disclosed unitary absorbent structures have improved performance over absorbent composites made of multiple separate components, and due to their unitary structure, offer significant simplification of the process of making personal hygiene articles.

In certain embodiments of the absorbent structure, the range of basis weight of the overall structure is from about 5 gsm to about 1000 gsm, or from about 5 gsm to about 600 gsm, or from about 10 gsm to about 400 gsm, or from about 20 gsm to 300 gsm, or from about 10 gsm to about 200 gsm, or from about 20 gsm to about 200 gsm, or from about 30 gsm to about 200 gsm, or from about 40 gsm to about 200 gsm. In certain embodiments where an absorbent core is present, the range of basis weight of the overall material can be from about 10 gsm to about 1500 gsm, or from about 50 gsm to about 1000 gsm, or from about 100 gsm to about 800 gsm.

The caliper refers to the caliper of the entire nonwoven material, inclusive of all layers, *i.e.,* the absorbent structure and the absorbent core, if present. In certain embodiments, the caliper of the material can range from about 0.1 mm to about 15.0 mm, or from about 0.2 mm to about 10.0 mm, or from about 0.5 mm to about 8.0 mm.

The presently disclosed absorbent structures can have improved fluid acquisition characteristics. For example, the absorbent structures can absorb a fluid with minimal runoff. In certain embodiments, runoff from the nonwoven materials will be less than about 40%, less than about 30%, less than about 20%, or less than about 10% of the original amount of fluid applied to the nonwoven material. A person having ordinary skill in the art will appreciate that the amount of runoff, as well as any other absorbency characteristics of a nonwoven material, can vary. For example, the observed absorbency characteristics can vary based on the amount of fluid and the surface area of the nonwoven material. Additionally, when the absorbent structure is layered with an absorbent core, the materials can have improved fluid acquisition characteristics. Furthermore, the absorbent structures of the presently disclosed subject matter can quickly absorb a fluid. In certain embodiments, an absorbent structure, as described above, can absorb a fluid in less than about 100 seconds, less than about 80 seconds, less than about 60 seconds, less than about 45 seconds, or less than about 30 seconds. The time it takes for a material to absorb a fluid can be called an "acquisition time." For example, and not limitation, the acquisition time can be measured using the procedures described in Comparative Example 1 and Example 5 below.

Furthermore, the presently disclosed absorbent structures can have improved dryness characteristics, indicating improved fluid retention. For example, after absorbing a fluid, the absorbent structures can be pressed to measure the amount of fluid released. In certain embodiments, when the absorbent material includes an absorbent core, a rewet test or a humidity sensation test can be used to press the absorbent structure and measure the released fluid, as described in Comparative Example 1 and Example 5 below. In certain embodiments, less than about 0.5 g, less than about 0.4 g, or less than about 0.3 g is released.

### Methods of Making the Materials

A variety of processes can be used to assemble the materials used in the practice of this disclosed subject matter to produce the unitary absorbent structures, including but not limited to, traditional dry forming processes such as airlaying and carding or other forming technologies such as spunlace or airlace. Preferably, the materials can be prepared by airlaid processes. Airlaid processes include, but are not limited to, the use of one or more forming heads to deposit raw materials of differing compositions in selected order in the manufacturing process to produce a product with distinct strata. This allows great versatility in the variety of products which can be produced.

In one embodiment, the material is prepared as a continuous airlaid web. The airlaid web is typically prepared by disintegrating or defiberizing a cellulose pulp sheet or sheets, typically by hammermill, to provide individualized fibers. Rather than a pulp sheet of virgin fiber, the hammermills or other disintegrators can be fed with recycled airlaid edge trimmings and off-specification transitional material produced during grade changes and other airlaid production waste. Being able to thereby recycle production waste would contribute to improved economics for the overall process. The individualized fibers from whichever source, virgin or recycled, are then air conveyed to forming heads on the airlaid web-forming machine. A number of manufacturers make airlaid web forming machines suitable for use in the disclosed subject matter, including Dan-Web Forming of Aarhus, Denmark, M&J Fibretech A/S of Horsens, Denmark, Rando Machine Corporation, Macedon, N.Y. which is described in U.S. Pat. No. 3,972,092, Margasa Textile Machinery of Cerdanyola del Valles, Spain, and DOA International of Wels, Austria. While these many forming machines differ in how the fiber is opened and air-conveyed to the forming wire, they all are capable of producing the webs of the presently disclosed subject matter. The Dan-Web forming heads include rotating or agitated perforated drums, which serve to maintain fiber separation until the fibers are pulled by vacuum onto a foraminous forming conveyor or forming wire. In the M&J machine, the forming head is basically a rotary agitator above a screen. The rotary agitator may comprise a series or cluster of rotating propellers or fan blades. Other fibers, such as a synthetic thermoplastic fiber, are opened, weighed, and mixed in a fiber dosing system such as a textile feeder supplied by Laroche S. A. of Cours-La Ville, France. From the textile feeder, the fibers are air conveyed to the forming heads of the airlaid machine where they are further mixed with the comminuted cellulose pulp fibers from the hammer mills and deposited on the continuously moving forming wire. Where defined layers are desired, separate forming heads may be used for each type of fiber. Alternatively or additionally, one or more layers can be prefabricated prior to being combined with additional layers, if any.

The airlaid web is transferred from the forming wire to a calendar or other densification stage to densify the web, if necessary, to increase its strength and control web thickness. In one embodiment, the fibers of the web are then bonded by passage through an oven set to a temperature high enough to fuse the included thermoplastic or other binder materials. In a further embodiment, secondary binding from the drying or curing of a latex spray or foam application occurs in the same oven. The oven can be a conventional through-air oven, be operated as a convection oven, or may achieve the necessary heating by infrared or even microwave irradiation. In particular embodiments, the airlaid web can be treated with additional additives before or after heat curing.

### Applications and End Uses

The absorbent structures of the disclosed subject matter can be used for any application known in the art. For example, the absorbent structures can be used either alone or as a component in a variety of absorbent articles. In certain aspects, the absorbent structures can be used in absorbent articles that absorb and retain body fluids. Such absorbent articles include baby diapers, adult incontinence products, sanitary napkins and the like.

### EXAMPLES

The following examples are merely illustrative of the presently disclosed subject matter and they should not be considered as limiting the scope of the subject matter in any way.

### COMPARATIVE EXAMPLE 1: Three-layer Absorbent Composite Material

For purpose of comparison, the present Example provides an absorbent composite made of three separate layers stacked vertically.

The top layer was a topsheet, the layer below the topsheet was a liquid acquisition layer, and the bottom layer was an absorbent core containing superabsorbent powder (SAP). This composite was made and tested for comparative purposes. The portion of the composite consisting of the topsheet and the liquid acquisition layer was designated "Control". The topsheet used in this Example was a commercial 10 gsm nonwoven spunbond material, Spunbico Hydrophilic IB4FZ-105 010 KG (Fitesa, Italy). The liquid acquisition layer was a commercial 60 gsm latex-bonded airlaid (LBAL) product, Vicell 6609 (Georgia-Pacific, Steinfurt, Germany). The absorbent core was a 175 gsm commercial multi-bonded airlaid (MBAL), 175 MBS3A, containing SAP (Georgia-Pacific, Steinfurt, Germany).

The liquid acquisition characteristics of the Control (the topsheet with the acquisition layer) were measured for a synthetic blood solution using the liquid acquisition performance testing procedures described below. The Control (the topsheet and the acquisition layer) was also placed on top of the absorbent core to form a three-layer absorbent composite. Synthetic blood was purchased from Johnson, Moen & Co. Inc. (Rochester, MN, Lot # 201141; February 2014). The synthetic blood had a surface tension of 40-44 dynes/cm (ASTM F23.40-F1670) and included various chemicals including ammonium polyacrylate polymer, Azo Red Dye, and HPLC distilled water, among other proprietary ingredients. The synthetic blood was diluted with deionized water to a composition of 35% blood and 65% water.

The testing apparatus consisted of an 11½ inch x 7½ inch x ¼ inch hard plastic plate with a ¾ inch inner diameter hole cut in the center (1 inch = 2.54 cm) A weighted stainless steel cylinder with a ¾ inch inner diameter was attached above the hole. The cylinder had a height of 2 inches, resulting in the complete apparatus having a total height of 2¼ inches and weight of 747.3 g.

The composite was compressed with an 8.190 kG plate for 1 minute and then was tested for the liquid acquisition performance of the Control (the topsheet and the acquisition layer) using the prepared synthetic blood solution. The composite was insulted with 4 mL of the synthetic blood at a rate of 10 mL/min. The acquisition time was measured from the start of insult until the liquid was no longer visible in the insult cylinder. A total of three insults were performed, yielding three acquisition times. The time interval between each of the three insults was 10 minutes. Table 1 illustrates the average acquisition times four samples of the Control.

Further, the rewet characteristics of each material were analyzed after measuring the three acquisition times. After the third acquisition time measurement, three pre-weighed square plies (4 inch by 4 inch) of collagen (Coffi collagen supplied by Viscofan USA), were placed on top of the tested composite. A thin Plexiglas plate and a weight were placed on top of the collagen plies for one minute. The Plexiglas and weight exerted a total pressure of 1.7 kPa. The collagen plies were weighed to determine the rewet result. Table 1 illustrates the rewet results of the tested composite.

**Table 1. Acquisition Times and Rewet Results of Control**

| **Trial** | **Time 1 (sec)** | **Time 2 (sec)** | **Time 3 (sec)** | **Rewet Wt. (g)** | **Total time (sec)** |
|---|---|---|---|---|---|
| 1 | 27.22 | 36.41 | 59.28 | 0.33 | -- |
| 2 | 27.57 | 37.47 | 64.15 | 0.31 | -- |
| 3 | 28.49 | 36.47 | 68.59 | 0.33 | -- |
| Avg. | 27.76 | 36.78 | 64.01 | 0.32 | 128.55 |

### EXAMPLE 1: Two-layer unitary structure with integrated top flowback barrier and liquid acquisition functions

This Example provides for a two-layer unitary structure having an integrated top flowback barrier and liquid acquisition functions. The top flowback barrier can be suitable for keeping the skin dry during the use of a hygiene product including the two-layer unitary structure by a consumer.

Structure A is a two-layer unitary structure which was formed using a laboratory pad former. 30 gsm of polyester (PET) fibers (Trevira Type 245, 6.7 dtex, 3 mm) were deposited on a 10 gsm nonwoven spunbond material, IB4FZ-105 010 KG (Fitesa, Italy). Both sides of the resulting two-layer structure were treated with 5 gsm polymeric binder (Vinnapas 192, Wacker) in the form of an emulsion and then cured in an air-through lab oven. Three samples of Structure A were prepared.

### EXAMPLE 2: Three-layer unitary structure with integrated top flowback barrier and liquid acquisition functions

This Example provides for a three-layer unitary structure having an integrated top flowback barrier and liquid acquisition functions.

Structure B was prepared as a three-layer unitary structure and formed using a laboratory pad former. 10 gsm eccentric bicomponent (bico) fibers (Fibervisions ESE430ALV1, 3.3 dtex, 4 mm) were deposited on a 10 gsm nonwoven spunbond material, Spunbico Hydrophilic IB4FZ-105 010 KG (Fitesa, Italy). Then, 20 gsm eccentric bicomponent fibers (Fibervisions ESE452ALV1, 5.7 dtex, 4 mm) were laid on top of the already formed layer of the bicomponent 5.7 dtex fibers. Thus formed three-layer webs were then cured in an air-through lab oven. Three samples of Structure B were prepared.

### EXAMPLE 3: Four-layer unitary structure with integrated top flowback barrier, liquid acquisition, and temporary storage functions

This Example provides four-layer unitary structures having an integrated top flowback barrier, liquid acquisition, and temporary storage functions. This Example includes two different unitary structures: Structures C and D.

Structure C is a four-layer structure that was made using a lab pad former. The formed four-layer webs were then cured in an air-through lab oven. Three samples of Structure C were prepared.

Structure D is a four-layer structure that was formed using a lab pad former. The top layer of Structure D was composed of 20 gsm bico fibers (Trevira Type 255, 1.7 dtex, 6 mm). The second layer of the structure was 20 gsm of PE/PP bicomponent fibers (Fibervisions, 5.7 dtex, 4 mm eccentric). Layer three was composed of 20 gsm PE/PP bicomponent fibers (Fibervisions, 3.3 dtex, 4 mm eccentric). The bottom layer was composed of 20 gsm of cellulose (GP 4725, semi treated pulp, Georgia-Pacific), which was bonded with a 5 gsm polymeric binder in the form of emulsion (Vinnapas 192, Wacker). The formed four-layer webs were then cured in an air-through lab oven. Three samples of Structure D were prepared.

### EXAMPLE 4: Multi-layer unitary structures having integrated top flowback barrier and liquid acquisition functions

This Example provides for five multi-layer unitary structures (Structures E, F, G, H, and I) having integrated top flowback barrier, liquid acquisition, and temporary storage functions. Each structure of this Example includes a blend of different bicomponent fibers in a top layer.

Structure E is a two-layer structure that was formed using a lab pad former. The top layer of Structure E was composed of a blend of 5 gsm bico fibers (Trevira Type 255, 1.7 dtex, 6 mm) and 15 gsm PE/PP bicomponent fibers (Fibervisions, 2.2 dtex, 4 mm eccentric). The bottom layer of the structure was 20 gsm of PE/PP bicomponent fibers (Fibervisions, 5.7 dtex, 6 mm eccentric). The formed two-layer webs were then cured in an air-through lab oven. Three samples of Structure E were prepared.

Structure F is a two-layer structure that was formed using a lab pad former. The top layer of Structure F was composed of a blend of 10 gsm bico fibers (Trevira Type 255, 1.7 dtex, 6 mm) and 10 gsm PE/PP bicomponent fibers (Fibervisions, 2.2 dtex, 4 mm eccentric). The bottom layer of the structure was 20 gsm of PE/PP bicomponent fibers (Fibervisions, 5.7 dtex, 6 mm eccentric). The formed two-layer webs were then cured in an air-through lab oven. Three samples of Structure F were prepared.

Structure G is a two-layer structure that was formed using a lab pad former. The top layer of Structure G was composed of a blend of 15 gsm bico fibers (Trevira Type 255, 1.7 dtex, 6 mm) and 5 gsm PE/PP bicomponent fibers (Fibervisions, 2.2 dtex, 4 mm eccentric). The bottom layer of the structure was 20 gsm of PE/PP bicomponent fibers (Fibervisions, 5.7 dtex, 6 mm eccentric). The formed two-layer webs were then cured in an air-through lab oven. Three samples of Structure G were prepared.

Structure H is a two-layer structure which was formed using a lab pad former. The top layer of Structure H was composed of a blend of 20 gsm bico fibers (Trevira Type 255, 1.7 dtex, 6 mm) and 5 gsm PE/PP bicomponent fibers (Fibervisions, 2.2 dtex, 4 mm eccentric). The bottom layer of the structure was 20 gsm of PE/PP bicomponent fibers (Fibervisions, 5.7 dtex, 6 mm eccentric). The formed two-layer webs were then cured in an air-through lab oven. Three samples of Structure H were prepared.

Structure I is a three-layer structure which was formed using a lab pad former. The top layer of Structure I was composed of a blend of 20 gsm bico fibers (Trevira Type 255, 1.7 dtex, 6 mm) and 5 gsm PE/PP bicomponent fibers (Fibervisions, 2.2 dtex, 4 mm eccentric). The middle layer was composed of 17 gsm of PE/PP bicomponent fibers (Fibervisions, 5.7 dtex, 6 mm eccentric) and the bottom layer of the structure was 8 gsm of PE/PP bicomponent fibers (Fibervisions, 3.3 dtex, 6 mm eccentric). Thus formed three-layer webs were then cured in an air-through lab oven. Three samples of Structure I were prepared.

### EXAMPLE 5: Liquid Acquisition and Rewet Measurements for Structures A-I

In this Example, the liquid acquisition characteristics of Structures A-I of Examples 1-4 were measured with the synthetic blood solution specified in Comparative Example 1. In each case, the tested Structure (A, B, C, D, E, F, G, H, or I) was placed on top of the absorbent core material described in Comparative Example 1. Thus, the absorbent core was, as in Comparative Example 1, a commercial 175 gsm commercial MBAL, 175 MBS3A, containing SAP (Georgia-Pacific, Steinfurt, Germany). The prepared composite materials made with Structures A-I and the absorbent core were compressed with an 8.190 kg plate for 1 minute. Then, the liquid acquisition test was conducted as described in Comparative Example 1. Table 2 summarizes the obtained acquisition time results.

Further, the rewet characteristics of each of Structures A, B, C, D, E, F, G, H, and I were analyzed after measuring the three acquisition times. The test procedure was the same as described in Comparative Example 1. Table 2 summarizes the obtained rewet results.

As shown in Table 2, the unitary multi-layer structures of Examples 1-4 had significantly improved acquisition times and rewet weight as compared to the Control (*see* Table 1). For example, for some structures, the total acquisition time was less than two-thirds that of the Control (128.55 sec.). Similarly, the rewet was also less than two-thirds that of the Control (0.32 g), and in some cases, less than one-half or less than one-third. Thus, the presently disclosed unitary absorbent structures have improved fluid acquisition and retention characteristics as compared to conventional composite materials.

**Table 2. Acquisition Times and Rewet Results of Structures A-I**

| **Structure** | **Trial** | **Time 1 (sec)** | **Time 2 (sec)** | **Time 3 (sec)** | **Rewet Wt. (g)** | **Total time (sec)** |
|---|---|---|---|---|---|---|
| A | 1 | 26.1 | 29.4 | 34.2 | 0.2 | -- |
| | 2 | 26.3 | 30.3 | 32.5 | 0.24 | -- |
| | 3 | 25.9 | 28.9 | 32.2 | 0.23 | -- |
| | Avg. | 26.1 | 29.5 | 33.0 | 0.22 | 88.6 |
| B | 1 | 26.2 | 30.1 | 31.7 | 0.2 | -- |
| | 2 | 25.6 | 30.7 | 30.8 | 0.17 | -- |
| | 3 | 26.0 | 28.5 | 28.9 | 0.17 | -- |
| | Avg. | 26.0 | 29.8 | 30.5 | 0.18 | 86.2 |
| c | 1 | 26.2 | 27.8 | 28.2 | 0.13 | -- |
| | 2 | 25.9 | 27.3 | 28.4 | 0.15 | -- |
| | 3 | 26.0 | 27.3 | 28.0 | 0.15 | -- |
| | Avg. | 26.0 | 27.5 | 28.2 | 0.14 | 81.7 |
| D | 1 | 25.5 | 34.3 | 49.2 | 0.13 | -- |
| | 2 | 25.8 | 28.8 | 43.8 | 0.1 | -- |
| | 3 | 25.6 | 33.1 | 45.1 | 0.15 | -- |
| | Avg. | 25.7 | 32.1 | 46.0 | 0.14 | 103.7 |
| E | 1 | 25.0 | 25.4 | 28.4 | 0.13 | -- |
| | 2 | 25.1 | 25.5 | 26.1 | 0.14 | -- |
| | 3 | 25.1 | 25.6 | 27.2 | 0.15 | -- |
| | Av. | 25.1 | 25.5 | 27.2 | 0.14 | 77.8 |
| F | 1 | 24.9 | 25.4 | 26.9 | 0.19 | -- |
| | 2 | 25.3 | 25.5 | 27.3 | 0.13 | -- |
| | 3 | 25.5 | 25.5 | 27.5 | 0.15 | -- |
| | Avg. | 25.2 | 25.5 | 27.2 | 0.16 | 77.9 |
| G | 1 | 25.21 | 25.29 | 30.06 | 0.15 | -- |
| | 2 | 25.4 | 26.31 | 27.22 | 0.17 | -- |
| | 3 | 25.09 | 25.35 | 27.33 | 0.12 | -- |
| | Avg. | 25.23 | 25.65 | 28.20 | 0.15 | 79.1 |
| H | 1 | 25.00 | 25.5 | 25.25 | 0.11 | -- |
| | 2 | 25.21 | 25.22 | 26.32 | 0.10 | -- |
| | 3 | 25.07 | 25.44 | 25.77 | 0.11 | -- |
| | Avg. | 25.09 | 25.39 | 25.78 | 0.11 | 76.3 |
| I | 1 | 25.09 | 25.32 | 25.53 | 0.07 | -- |
| | 2 | 24.97 | 25.25 | 25.44 | 0.09 | -- |
| | 3 | 25.14 | 25.25 | 25.69 | 0.10 | -- |
| | Avg. | 25.07 | 25.27 | 25.55 | 0.09 | 75.9 |

In addition to the various embodiments depicted and claimed, the disclosed subject matter is also directed to other embodiments having other combinations of the features disclosed and claimed herein. As such, the particular features presented herein can be combined with each other in other manners within the scope of the disclosed subject matter such that the disclosed subject matter includes any suitable combination of the features disclosed herein. The foregoing description of specific embodiments of the disclosed subject matter has been presented for purposes of illustration and description. It is not intended to be exhaustive or to limit the disclosed subject matter to those embodiments disclosed.

It will be apparent to those skilled in the art that various modifications and variations can be made in the systems and methods of the disclosed subject matter without departing from the scope of the disclosed subject matter. Thus, it is intended that the disclosed subject matter include modifications and variations that are within the scope of the appended claims.

## Claims

1. A multi-layer unitary absorbent structure, comprising:
a first layer comprising a blend of different first and second bicomponent fibers and having a basis weight from about 5 gsm to about 50 gsm, wherein the blend of bicomponent fibers comprises from 50 to 100 percent by weight of the layer; and
a second layer, adjacent to the first layer, comprising a third type of bicomponent fibers and having a basis weight from about 5 gsm to about 50 gsm, and wherein the bicomponent fibers of the second layer have a greater dtex value than the bicomponent fibers of the blend forming thefirst layer and comprise from 50 to 100 percent by weight of the layer.

2. The multi-layer unitary absorbent structure of claim 1, further comprising a third layer, adjacent to the second layer, comprising a fourth type of bicomponent fiber.

3. The multi-layer unitary absorbent structure of claim 1, wherein the first and second bicomponent fibers differ by at least one of composition, configuration, dtex, and length.

4. The multi-layer unitary absorbent structure of claim 1, further comprising a cellulosic fiber layer adjacent to the second layer.

5. The multi-layer unitary absorbent structure of claim 1, further comprising an absorbent core.

6. The multi-layer unitary absorbent structure of claim 5, wherein the absorbent core comprises SAP.

7. The multi-layer unitary absorbent structure of claim 1, further comprising a binder on at least a portion of the external surface of at least one outer layer.

8. The multi-layer unitary absorbent structure of claim 1, wherein the first and second bicomponent fibers are present in a ratio of about 5:1 to about 1:5.

9. The multi-layer unitary absorbent structure of claim 2, wherein the bicomponent fibers of the second and third layers have a greater dtex than the bicomponent fibers of the blend forming the first layer.

10. An absorbent article comprising the multi-layer unitary absorbent structure of any one of claims 1-9.

11. The absorbent article of claim 10, wherein the absorbent article is a hygiene product.

12. The absorbent article of claim 11, wherein the hygiene product is selected from a baby diaper, an adult incontinence product, and a sanitary napkin.

## Patentansprüche

1. Mehrschichtige, einheitliche, absorbierende Struktur, umfassend:
eine erste Schicht, die eine Mischung aus verschiedenen ersten und zweiten Bikomponentenfasern umfasst und ein Flächengewicht von etwa 5 g/m² bis etwa 50 g/m² aufweist, wobei die Mischung aus Bikomponentenfasern 50 bis 100 Gewichtsprozent der Schicht umfasst; und
eine zweite, an die erste Schicht angrenzende Schicht, die eine dritte Art von Bikomponentenfasern umfasst und ein Flächengewicht von etwa 5 g/m² bis etwa 50 g/m² aufweist, und wobei die Bikomponentenfasern der zweiten Schicht einen höheren dtex-Wert aufweisen als die Bikomponentenfasern der die erste Schicht bildenden Mischung und 50 bis 100 Gewichtsprozent der Schicht ausmachen.

2. Die mehrschichtige, einheitliche, absorbierende Struktur nach Anspruch 1, die ferner eine dritte, an die zweite Schicht angrenzende Schicht umfasst, die eine vierte Art von Bikomponentenfasern umfasst.

3. Die mehrschichtige, einheitliche, absorbierende Struktur nach Anspruch 1, wobei sich die erste und die zweite Bikomponentenfaser durch mindestens eines aus Zusammensetzung, Konfiguration, dtex und Länge unterscheiden.

4. Die mehrschichtige, einheitliche, absorbierende Struktur nach Anspruch 1, die ferner eine an die zweite Schicht angrenzende Cellulosefaserschicht umfasst.

5. Die mehrschichtige, einheitliche, absorbierende Struktur nach Anspruch 1, die ferner einen absorbierenden Kern umfasst.

6. Die mehrschichtige, einheitliche, absorbierende Struktur nach Anspruch 5, wobei der absorbierende Kern SAP umfasst.

7. Die mehrschichtige, einheitliche, absorbierende Struktur nach Anspruch 1, die weiterhin ein Bindemittel auf mindestens einem Teil der äußeren Oberfläche mindestens einer äußeren Schicht umfasst.

8. Die mehrschichtige, einheitliche, absorbierende Struktur nach Anspruch 1, wobei die ersten und zweiten Bikomponentenfasern in einem Verhältnis von etwa 5:1 bis etwa 1:5 vorhanden sind.

9. Die mehrschichtige, einheitliche, absorbierende Struktur nach Anspruch 2, wobei die Bikomponentenfasern der zweiten und dritten Schichten einen größeren dtex aufweisen als die Bikomponentenfasern der Mischung, die die erste Schicht bildet.

10. Absorbierender Artikel, umfassend die mehrschichtige, einheitliche, absorbierende Struktur nach einem der Ansprüche 1 bis 9.

11. Der absorbierende Artikel nach Anspruch 10, wobei der absorbierende Artikel ein Hygieneprodukt ist.

12. Der absorbierende Artikel nach Anspruch 11, wobei das Hygieneprodukt ausgewählt ist aus einer Babywindel, einem Inkontinenzprodukt für Erwachsene und einer Damenbinde.

## Revendications

1. Structure absorbante unitaire multicouche, comprenant :
une première couche comprenant un mélange de premières et secondes fibres bicomposées différentes et ayant un poids de base d'environ 5 g/m² à environ 50 g/m², le mélange des fibres bicomposées comprenant de 50 à 100 pour cent en poids de la couche ; et
une seconde couche, adjacente à la première couche, comprenant un troisième type de fibres bicomposées et ayant un poids de base d'environ 5 g/m² à environ 50 g/m², et les fibres bicomposées de la seconde couche ayant une valeur dtex supérieure à celle des fibres bicomposées du mélange formant la première couche et comprenant de 50 à 100 pour cent en poids de la couche.

2. Structure absorbante unitaire multicouche selon la revendication 1, comprenant en outre une troisième couche, adjacente à la seconde couche, comprenant un quatrième type de fibre bicomposée.

3. Structure absorbante unitaire multicouche selon la revendication 1, les premières et secondes fibres bicomposées différant d'au moins l'une parmi la composition, la configuration, la valeur dtex, et la longueur.

4. Structure absorbante unitaire multicouche selon la revendication 1, comprenant en outre une couche de fibre cellulosique adjacente à la seconde couche.

5. Structure absorbante unitaire multicouche selon la revendication 1, comprenant en outre un cœur absorbant.

6. Structure absorbante unitaire multicouche selon la revendication 5, le cœur absorbant comprenant du SAP.

7. Structure absorbante unitaire multicouche selon la revendication 1, comprenant en outre un agent liant sur au moins une portion de la surface externe d'au moins une couche externe.

8. Structure absorbante unitaire multicouche selon la revendication 1, les premières et secondes fibres bicomposées étant présentes sous un rapport d'environ 5:1 à environ 1:5.

9. Structure absorbante unitaire multicouche selon la revendication 2, les fibres bicomposées des seconde et troisième couche ayant un dtex supérieur à celui des fibres bicomposées du mélange formant la première couche.

10. Article absorbant comprenant la structure absorbante unitaire multicouche selon l'une quelconque des revendications 1 à 9.

11. Article absorbant selon la revendication 10, l'article absorbant étant un produit d'hygiène.

12. Article absorbant selon la revendication 11, le produit d'hygiène étant sélectionné parmi une couche pour bébé, un produit d'incontinence pour adulte, et une serviette hygiénique.
